# EUROPEAN PATENT APPLICATION

(11) **EP 2 594 234 A2**
(43) Date of publication of application: **22.05.2013**
(21) Application number: 12197210.3
(22) Date of filing: 15.04.2009
(51) Int. Cl.: A61F 7/00, A61G 7/00

(54) **Microclimate management system**

(30) Priority: 15.04.2008 US 45111 P
(62) Divisional of application: 09733293.6
(71) Applicant: Hill-Rom Services, Inc., Batesville, IN 47006-9167 (US)
(72) Inventor: Lachenbruch, Charles A., Lakeway, TX Texas 78734 (US); Douglas, Stephen, L., Batesville, IN Indiana 47006 (US); Flint, Stephen, C., Fortville, IN Indiana 46040 (US); Williamson, Rachel, Osgood, IN Indiana 47037 (US)
(74) Representative: Findlay, Alice Rosemary

(57) **Abstract**

A microclimate management system 10 comprises a mattress 16, a topper 18, and a control system 14. The topper 18 is coupled to the mattress 16 and is supported thereon. The topper 18 defines an interior region 42 and a person contacting surface 52. The control system 14 is configured to maintain at least one of a surface temperature, a relative surface humidity, and a heat withdrawal capacity of at least a portion of the person contacting surface 52 within a predetermined operating range.

## Description

### CROSS REFERENCE TO RELATED APPLICATIONS

This Application claims priority to U.S. Provisional Application Serial No. 61/045,111 titled MICROCLIMATE MANAGEMENT SYSTEM filed on April 15, 2008, the contents of which are incorporated herein by reference.

### BACKGROUND

This disclosure relates to microclimate management systems, and more particularly, but not exclusively to microclimate management systems adapted to maintain the temperature and/or relative humidity of a patient contacting surface of a support device within a predetermined range in some exemplary embodiments.

Patients lying on support devices, such as hospital bed mattresses, for extended periods of time are susceptible to the development of pressure ulcers (also known as decubitus ulcers or bedsores). Pressure ulcers are lesions often found adjacent bony or cartilaginous areas. Pressure ulcers may be caused by tissue forces, such as, for example, pressure, i.e., compression of tissues, shear force, and friction. Pressure ulcer formation may be exacerbated by the presence of excess body heat and/or moisture.

While various microclimate management systems have been developed, in certain applications there is still room for improvement. Thus, a need persists for further contributions in this area of technology.

### SUMMARY

One illustrative embodiment, a microclimate management system including a support device, a fluid supply, and a controller having an instruction set that causes the controller to regulate the rate and temperature of fluid supplied by the fluid supply to maintain a heat withdrawal capacity of a least a portion of the person contacting surface below about 140W/m². In another illustrative embodiment, a microclimate management system can include a mattress, a topper supported on the mattress, and a control system configured to maintain at least one of the surface temperature, humidity, and heat withdrawal capacity of at least a portion of the person contacting surface within a predetermined range. In yet another illustrative embodiment, a microclimate management system including a support device, a fluid supply, and a controller having an instruction set that causes the controller to regulate the rate and temperature of fluid supplied by the fluid supply to maintain a surface temperature of at least a portion of the person contacting surface above 88 °F and a relative humidity of at least a portion of the person contacting surface below about 95%. In still another illustrative embodiment, a fluid supply in communication with a topper is regulated as a function of at least one of the temperature and the relative humidity of at least a portion of the person contacting surface to maintain at least a portion of the person contacting surface within a predetermined operating range. In a further illustrative embodiment, a fluid supply in communication with a support device is regulated as a function of at least two of the temperature and the relative humidity of at least a portion of the person contacting surface and a user input to maintain at least a portion of the person contacting surface within a predetermined operating range. In yet a further illustrative embodiment, a fluid supply in communication with a topper is regulated as a function of at least one of the temperature and the relative humidity of at least a portion of the person contacting surface, and a user input to maintain at least a portion of the person contacting surface within a predetermined operating range. In still a further illustrative embodiment, at least one of a rate and a temperature of a fluid supplied by a fluid supply to a support device is regulated as a function of the temperature and relative humidity of the person contacting surface to maintain the person contacting surface within a predetermined operating range. In another illustrative embodiment, an apparatus includes a mattress, a topper, and a sensor configured to sense at least one of a temperature or a person contacting surface, a relative humidity of a person contacting surface, a temperature of the fluid within the topper, and a relative humidity of the fluid within the topper. In yet another illustrative embodiment, a fluid supply is selected to cooperate with at least one of the fluid permeability parameter of the cover, the thickness parameter of the cover, the thermal conductivity of the cover, the fluid resistance parameter of the spacer, the fluid permeability parameter of the spacer, the thermal conductivity parameter of the spacer, and the thickness parameter of the spacer to maintain a heat withdrawal capacity of at least a portion of the person contacting surface below about 140 W/m². In still another embodiment, at least one of the fluid permeability parameter of the cover, the thickness parameter of the cover, the thermal conductivity of the cover, the fluid resistance parameter of the spacer, the fluid permeability parameter of the spacer, the thermal conductivity parameter of the spacer, and the thickness parameter of the spacer is varied to cooperate with the fluid supply to maintain a heat withdrawal capacity of at least a portion of the person contacting surface below about 140 W/m².

Further embodiments of this disclosure will become apparent from the following description and accompanying drawings included herewithin.

### BRIEF DESCRIPTION OF THE DRAWINGS

FIG. 1 is a graph illustrating the relationship between the pressure exerted on the skin and the amount of time the pressure is exerted before damage to the skin occurs for a given skin temperature.

FIG. 2 is a graph illustrating some principles of the present invention.

FIG. 3 is a perspective view of a microclimate management system according to one embodiment of the current disclosure including a support device and a control system.

FIG. 4 is a partial sectional view of the embodiment of FIG. 3 illustrating the controller and sensors positioned within a cover of the support device.

FIG. 5 is a cross-sectional side view of the illustrative support device of FIG. 3 including a low air-loss topper positioned on top of the upper mattress surface of the mattress.

FIG. 6 is a graph illustrating the relationship between the composition of the support device of FIG. 3 and the temperature of the skin contacting the support device over time, according to some principles of the present invention.

FIG. 7 is a partial cross-sectional side view of the illustrative support device of FIG. 3 taken along line 5-5 with a person resting thereon illustrating the flow of air, heat, and moisture through the support device.

FIG. 8 is a cross-sectional side view of a support device taken along line 5-5 of FIG. 3 according to another embodiment of the current disclosure including a low air-loss topper with bladders or a mattress with bladders.

FIG. 9 is a cross-sectional side view of a support taken along line 5-5 of FIG. 3 according to another embodiment of the current disclosure including a low air-loss topper positioned within the mattress cover mattress.

### DESCRIPTION OF SPECIFIC ILLUSTRATIVE EMBODIMENTS

While the system present in this disclosure can take many different forms, for the purpose of promoting an understanding of the disclosure, reference will now be made to the embodiments illustrated in the drawings, and specific language will be used to describe the same. No limitation of the scope of the disclosure is thereby intended. Alterations, further modifications of the described embodiments, and any further applications of the principles of the disclosure as described herein as would normally occur to one skilled in the art to which the disclosure relates are contemplated.

One illustrative embodiment, a microclimate management system including a support device, a fluid supply, and a controller having an instruction set that causes the controller to regulate the rate and temperature of fluid supplied by the fluid supply to maintain a heat withdrawal capacity of a least a portion of the person contacting surface below about 140W/m². In another illustrative embodiment, a microclimate management system can include a mattress, a topper supported on the mattress, and a control system configured to maintain at least one of the surface temperature, humidity, and heat withdrawal capacity of at least a portion of the person contacting surface within a predetermined range. In yet another illustrative embodiment, a microclimate management system including a support device, a fluid supply, and a controller having an instruction set that causes the controller to regulate the rate and temperature of fluid supplied by the fluid supply to maintain a surface temperature of at least a portion of the person contacting surface above 88 °F and a relative humidity of at least a portion of the person contacting surface below about 95%. In still another illustrative embodiment, a fluid supply in communication with a topper is regulated as a function of at least one of the temperature and the relative humidity of at least a portion of the person contacting surface to maintain at least a portion of the person contacting surface within a predetermined operating range. In a further illustrative embodiment, a fluid supply in communication with a support device is regulated as a function of at least two of the temperature and the relative humidity of at least a portion of the person contacting surface and a user input to maintain at least a portion of the person contacting surface within a predetermined operating range. In yet a further illustrative embodiment, a fluid supply in communication with a topper is regulated as a function of at least one of the temperature and the relative humidity of at least a portion of the person contacting surface, and a user input to maintain at least a portion of the person contacting surface within a predetermined operating range. In still a further illustrative embodiment, at least one of a rate and a temperature of a fluid supplied by a fluid supply to a support device is regulated as a function of the temperature and relative humidity of the person contacting surface to maintain the person contacting surface within a predetermined operating range. In another illustrative embodiment, an apparatus includes a mattress, a topper, and a sensor configured to sense at least one of a temperature or a person contacting surface, a relative humidity of a person contacting surface, a temperature of the fluid within the topper, and a relative humidity of the fluid within the topper. In yet another illustrative embodiment, a fluid supply is selected to cooperate with at least one of the fluid permeability parameter of the cover, the thickness parameter of the cover, the thermal conductivity of the cover, the fluid resistance parameter of the spacer, the fluid permeability parameter of the spacer, the thermal conductivity parameter of the spacer, and the thickness parameter of the spacer to maintain a heat withdrawal capacity of at least a portion of the person contacting surface below about 140 W/m². In still another embodiment, at least one of the fluid permeability parameter of the cover, the thickness parameter of the cover, the thermal conductivity of the cover, the fluid resistance parameter of the spacer, the fluid permeability parameter of the spacer, the thermal conductivity parameter of the spacer, and the thickness parameter of the spacer is varied to cooperate with the fluid supply to maintain a heat withdrawal capacity of at least a portion of the person contacting surface below about 140 W/m²

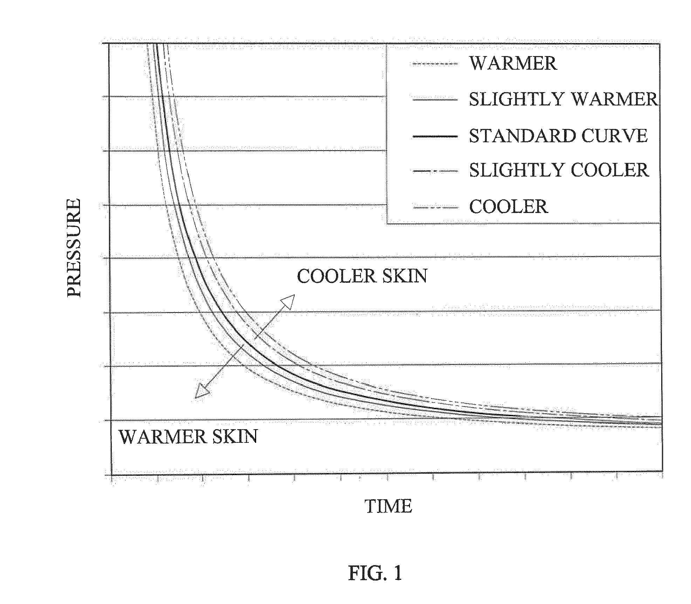

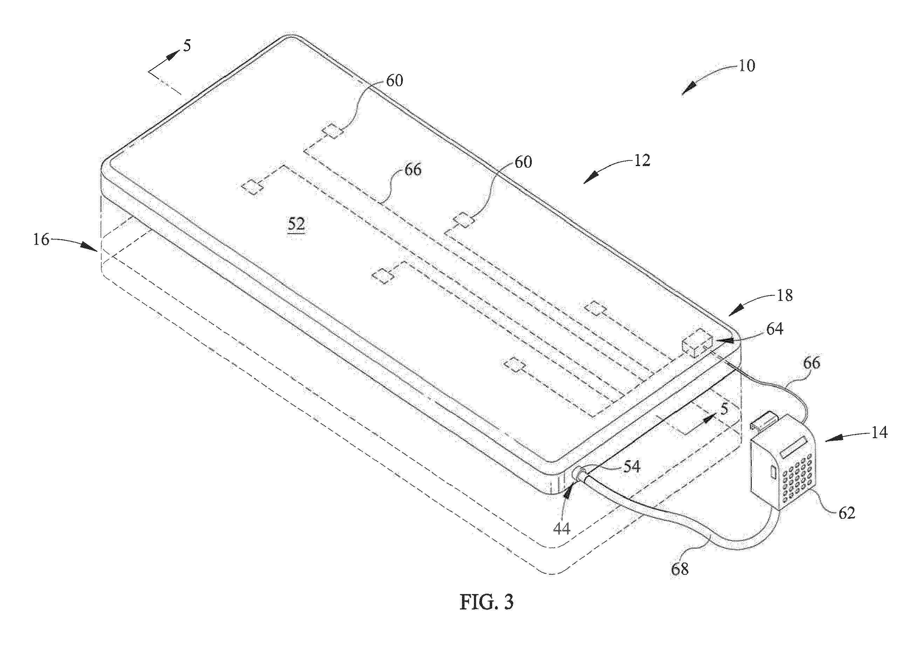

A microclimate management system 10, described in more detail in connection with FIG. 3, can manage the temperature of a person's skin. Limiting skin warming can be accomplished by cooling the skin, which can reduces the metabolic demand of the tissue so that the tissue can withstand an exerted pressure for a longer period without breaking down, as shown in FIG. 1. Cooling the skin to the point of excessive patient discomfort or hypothermia is preferably avoided.

A microclimate management system 10 can also manage the accumulation of moisture against a person's skin. By limiting skin warming and moisture accumulation, ischemia, which is a restriction in blood supply generally due to factors in the blood vessels with resultant damage or dysfunction of tissue, and maceration, which is the softening and weakening of the skin that occurs when its collagen linkages are dissolved over time, can be reduced. Furthermore, limiting moisture accumulation can help decrease the coefficient of friction between the wet skin and common bedding materials, thereby reducing the forces imposed on the weakened skin for any sliding movement.

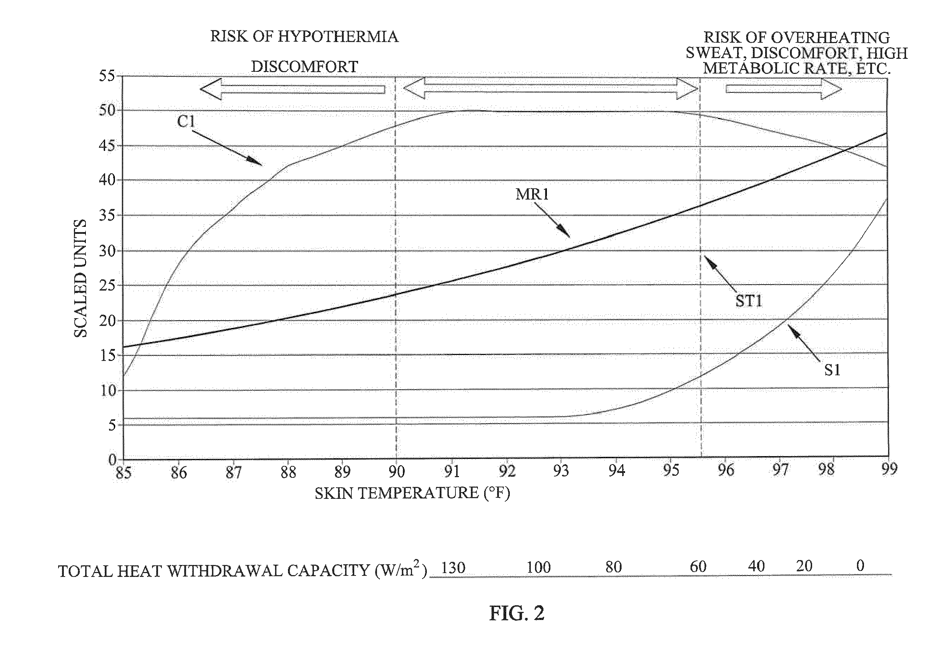

Skin temperature can influence the perspiration rate or sweat rate of the skin, metabolic demand of the skin, and person's level of comfort, as shown in FIG. 2, in which the x-axis corresponds to the temperature of the skin increasing from left to right (in degrees Fahrenheit) and the y-axis corresponds to scaled units representing the various dependent variables depicted. Curve S1, the sweat curve, illustrates the rate at which sweat is produced by the skin as the skin temperature varies. The sweat rate of the skin at a given location can be affected by the core temperature of the person, the person's mean skin temperature, and the local skin temperature. The sweat rate for a given patch of skin can increase as the patch of skin is warmed. The skin sweat rate can remain relatively constant at temperatures below a sweat threshold ST1 of approximately 35.3 °C (95.5 °F). The sweat rate below this threshold can be between about 5 g/m²-hr to 10 g/m²-hr. As the skin temperature increases beyond the sweat threshold the sweat rate can begin to rise dramatically.

Curve MR1, shown in FIG. 2, illustrates the extent to which the metabolic demand of the skin varies with the skin temperature. As the temperature of the skin increases, the demand for additional blood to be supplied to the skin can increases and the affected blood vessels can dilate to accommodate the demand. However, when high pressures are applied to the skin, dilation of the blood vessels can be impeded and nutrients may not be supplied to the skin at the necessary rate. This could result in a nutrient deficit leading to ischemic necrosis.

Comfort curve C1 illustrates the level of patient comfort experienced while resting on the microclimate management system 10 as a function of the skin temperature. While a patient's comfort level is not an exact measurement and can differ from person to person for any given temperature, the comfort curve is generally representative of the comfort level for the majority of patients observed. Variations in the level of comfort can be due to factors including, but not limited to, the person's body mass index, age, physical conditioning, personal preferences, and injury or illness.

Analysis of the sweat curve S1, metabolic rate curve MR1, and comfort curve C1, as shown in FIG. 2, demonstrates a range of skin temperatures among which the risk of pressure ulcers can be decreased. Specifically, a person's skin can be maintained in one embodiment at a temperature below approximately 35.8 °C (96.5 °F), a temperature at which comfort begins to noticeably decline, as shown by curve C1 in FIG. 2. More specifically, a person's skin can be maintained at a temperature below approximately 35.3 °C (95.5 °F), a temperature at which the sweat threshold ST1 is reached. Still more specifically, a person's skin can be maintained within a temperature range of about 32.2 °C (90 °F) and 35.3 °C (95.5 °F). In other embodiments, the temperature of the skin can be maintained between about 31.1 °C (88 °F) and 35.8 °C (96.5 °F). It should be appreciated that the temperature of the skin can be maintained below 32.2 °C (90 °F); however, the risk of causing the person resting on the microclimate management system 10 to go into hypothermia increases as the temperature of the patient the skin decreases. It should also be appreciated that the temperature of the skin can be maintained above 35.8 °C (96.5 °F); however, the risk of increasing the occurrences of pressure ulcers increases as the temperature of the skin increases.

The temperature of the skin can be maintained within the previously stated ranges for a minimum of 6 hours. More specifically, the temperature of the skin can be maintained within the previously stated temperature ranges for 12 hours or more. Still more specifically, the temperature of the skin can be maintained within the previously stated temperature ranges for 24 hours or more. In other embodiments the temperature of the skin can be maintained for less than 6 hours. It should be appreciated that other parameters, such as relative humidity and heat transfer rate, can be maintained within their respective ranges for the previously stated amounts of time.

The temperature of the skin of a person on the microclimate management system 10 can depend upon the heat withdrawn from their skin. The total heat withdrawal capacity (THW capacity) parameter is the cooling power of the surface in Watts/m² with higher values indicating greater heat transfer. The THW capacity depends upon both the heat withdrawn due to dry flux and that removed due to wet flux.

The dry flux measures the ability of a surface, such as the patient contacting surface 52 of the support apparatus 12 shown in FIG. 3, to remove heat from the person's skin in the absence of moisture on the skin. The dry flux remains constant regardless of how much the skin is sweating.

The wet flux represents the heat removed from the skin due to evaporation and is proportional to the evaporative capacity of a surface, such as the patient contacting surface 52 of the support apparatus 12 shown in FIG. 3, interfacing with the skin. The evaporative capacity measures a surface's ability to keep the skin relatively dry and is a measure of the surface's ability to promote evaporation. The parameter is measured in g/m²-hr with higher values again indicating better performance.

To maintain the skin of a typical person engaging a patient support 12, as shown in FIG. 3, between about 32.2 °C (90 °F) and about 35.3 °C (95.5 °F), the support device 12 and material (not shown) positioned between the person and the support device 12 can have a THW capacity of between about 60 W/m² and 125 W/m². In other embodiments, the TWH capacity can be maintained between about 50 W/m² and 140 W/m². It should be appreciated that the THW capacity can be maintained below 60 W/m²; however, the risk of increasing occurrences of pressure ulcers increases as the THW capacity increases. It should also be appreciated that the THW capacity can be maintained above 125 W/m²; however, the risk of causing the person to go into hypothermia increases as the THW capacity decreases.

The relative humidity of the skin of a person on the microclimate management system 10 can depend upon the heat removed from their skin due to evaporation. To maintain the skin of a typical person engaging a patient support 12 shown in FIG. 3 between about 32.2 °C (90 °F) and 35 °C (95 °F), the support device 12 can have a relative humidity ("RH") between about 55% RH and 90% RH. In other embodiments, the relative humidity range can be maintained between about 20% RH and about 95% RH. It should be appreciated that the relative humidity can be maintained below 55% RH; however, the risk of drying the skin excessively and dehydrating the person increases as the relative humidity decreases. It should also be appreciated that the relative humidity can be maintained above 90% RH; however, the risk of saturating the skin and increasing the occurrences of pressure ulcers increases as the relative humidity increases.

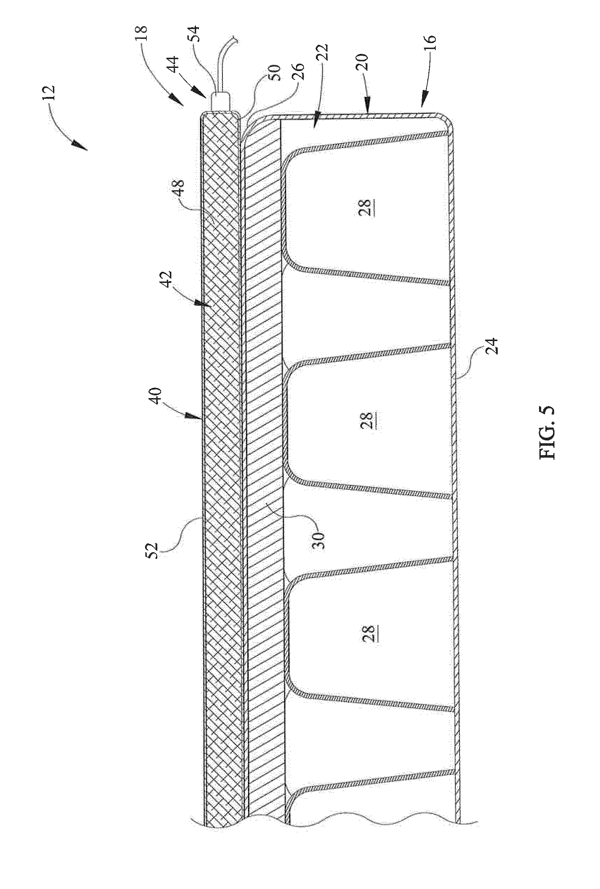

The microclimate management system 10, according to one embodiment of the current disclosure, can be configured to maintain the skin about the aforementioned ranges as shown in FIGs. 3-5. The microclimate management system 10 can include a support device 12 and a control system 14 adapted to manage the microclimate of the support device 12. The microclimate management system 10 can generally be used in the hospital setting on a hospital bed (not shown), stretcher (not shown), or other support structure to prevent the occurrences of pressure ulcers. It should be appreciated that the microclimate management system 10 can be used in any situation where a support device 12 is commonly used. The microclimate management system 10 can prevent the occurrences of pressure ulcers by reducing the accumulation of heat and moisture that tends to occur on a person's skin when the person is laid on the support device 12 in the supine position.

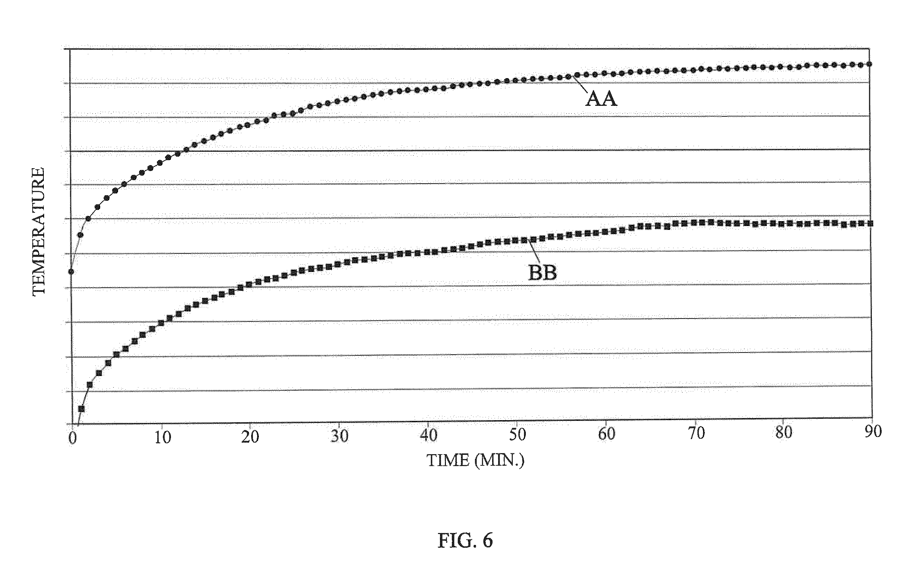

Some of the types of support devices 12 utilized within the hospital setting today are: foam support devices and low air-loss support devices. The difference in skin temperature over time as the skin remains in contact with the foam support devices is illustrated by curve AA, and difference in skin temperature over time as the skin remains in contact with the low air-loss support devices is illustrated by curve BB, as shown in FIG. 6. Foam support devices 12 can block the natural, basal level of heat and moisture produced by the skin that would normally flow into the atmosphere, whereas low air-loss support devices 12 can be adapted to remove accumulated heat and moisture. Blocking the heat and moisture produced warms and wets the skin over time, creating an environment that can differ markedly from the environment in which the skin was designed to operate.

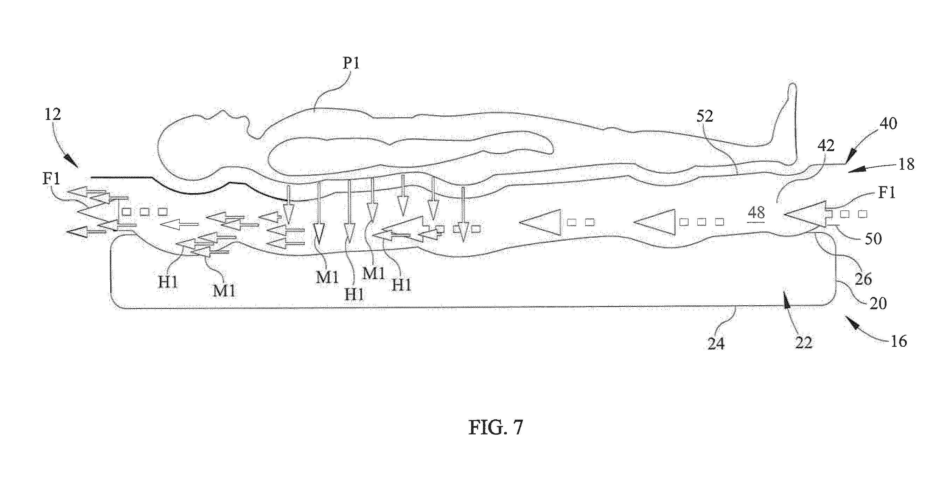

Low air-loss broadly refers to a feature of a support surface that provides a flow of air to assist in managing the heat and humidity (microclimate) of the skin. Two types of mechanisms that low air-loss support devices 12 typically use to remove accumulated moisture and heat: convective evaporation and diffusive evaporation. Convective evaporation evaporates accumulated moisture by blowing air on the skin. Diffusive evaporation evaporates accumulated moisture through and under the surface of the support device 12 to cool the skin without blowing air directly thereon. An example of a diffusive device can be seen in FIG. 7 where the air-loss support device 12 according to one embodiment of the current disclosure where the patient P1 is lying on the support device 12 in the supine position with fluid F1 flowing through the support device 12 to remove heat H1 and moisture M1 radiated by the patient P1. It should be appreciated that in some low air-loss support devices 12 a combination of diffusive and convective evaporation can be utilized.

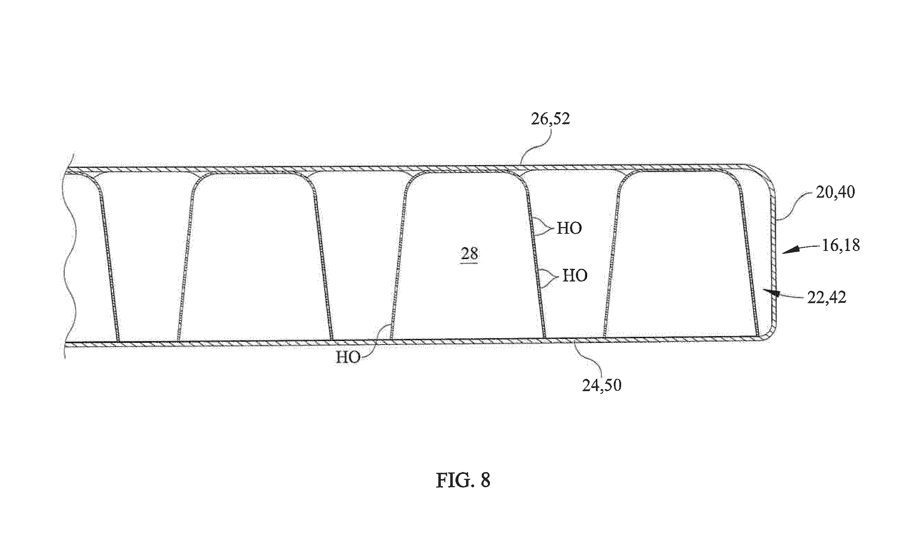

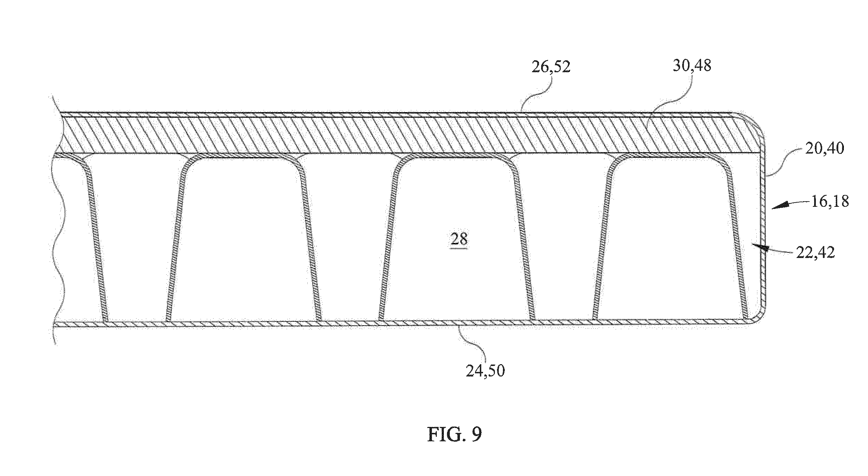

In one illustrative embodiment, the support device 12 can include a mattress 16 and a topper 18 as shown in FIG. 3. The topper 18 can be positioned on top of the mattress 16 and can be removably coupled to the mattress 16 by a plurality of fasteners (not shown), such as, buttons, snaps, Velcro®, ties, pins, zippers, or other known fasteners, to prevent movement of the topper 18 with respect to the mattress 16. It should be appreciated that the topper 18 can be integrally incorporated in the mattress 16 or can be configured to mimic the structure of the mattress 16 as shown in FIGs. 8 & 9.

The mattress 16 can include an outer mattress cover 20 or mattress ticking 20 that can define a mattress chamber 22. The mattress ticking 20 can have a lower mattress surface 24 that can interface with the hospital bed (not shown), and an upper mattress surface 26 that can interface with the LAL topper 18. The mattress chamber 22 can contain a plurality of air mattress bladders 28 and a mattress spacer 30 therein. It should be appreciated that the mattress 18 can contain only one mattress bladder 28 within the mattress chamber 22. It should also be appreciated that the mattress 18 can be composed of a polymeric material, such as, foam, or a combination of polymeric material and bladders. The mattress bladders 28 can be alternately inflated and deflated to create a form of alternating pressure therapy. The bladders 28 can also be in communication with one another such that the fluid pressure in the bladders 28 can be maintained as pressure exerted on the bladders 28. In other embodiments, the bladders 28 can include holes HO therein that allow for fluid therein to be communicated from the bladders 28 into the mattress chamber 22 as shown in FIG. 8. In still other embodiments, the bladders 28 can include other components, such as, bladders 28, for assisting with the turning of a patient, for assisting with lateral rotation of a patient, or other therapy or support bladders 28.

In one illustrative embodiment, the LAL topper 18 can be a low air-loss topper 18 (LAL topper 18) that can include an outer LAL cover 40 or LAL ticking 40 that can define a LAL chamber 42, an inlet 44, a vent 46, and a three-dimensionally engineered spacer 48. The LAL ticking 40 can include a lower LAL surface 50 and an upper LAL surface 52 or patient contacting surface 52. The lower LAL surface 50 can interface with the upper mattress surface 28. The patient contacting surface 52 can interface with a patient P1 lying thereon as shown in FIG. 7. It should be appreciated that the patient P1 can be separated from the patient contacting surface 52 by material (not shown), such as bedding, clothing, or other such garments or materials, that has a total thermal conductivity of anywhere from about .02 W/m - K to 1000 W/m - K. The lower LAL surface 50 and the patient contacting surface 52 can be generally shaped to cooperate with the upper mattress surface 26 and can be secured to one another along their respective edges by ultrasonic welding in order to form a fluid-tight seal. It should be appreciated that the lower LAL surface 50 and the patient contacting surface 52 can be secured to one another by adhesive, plastic welding, or other securing means, and can be secured along various portions of the lower LAL surface 50 and the patient contacting surface 52.

The LAL ticking 40 of this illustrative embodiment can be moisture permeable and air impermeable. It should be appreciated that the LAL ticking 40 can be both moisture permeable and air permeable. It should also be appreciated that the mattress ticking 20 and the LAL ticking 40 can be composed of the same material and have the same physical characteristics. The moisture vapor transfer rate for the LAL ticking 40, when fluid is being supplied to the LAL topper 18 at a rate of 2.2 ft³/min., can be at least about 20 g/m²-hr in order to accommodate basal skin moisture production. The moisture vapor transfer rate of the LAL ticking 40 can be 80 g/m²-hr or more. It should be appreciated that the moisture vapor transfer rate can be between 25 g/m²-hr and 200 g/m²-hr. It should be appreciated that the moisture vapor transfer rate can be less than about 20 g/m²-hr; however, the amount of moisture accumulation on the skin can increase with time and can increase the risk of maceration.

The inlet 44 can be positioned along a side of the LAL topper 18 and can allow for fluid to be communicated from the control system 14 into the LAL chamber 42. The inlet 44 can include an inlet coupler 54 that can couple the control system 14 to the LAL topper 18. It should be appreciated that the inlet coupler 54 can be secured to the LAL topper 18 such that there is a fluid tight seal between the inlet coupler 54 and the LAL topper 18.

The vent 46 or outlet 46 can be positioned along a side of the LAL topper 18 opposite the inlet 44. The vent 46 can be an opening in the lower LAL surface 50 that can allow fluid entering the inlet 44 and passing through the LAL chamber 42 to exit the LAL topper 18. It should be appreciated that the vent 46 can be a portion of the edges of the lower LAL surface 50 and the patient contacting surface 52 that were not secured to one another. It should also be appreciated that the vent 46 can be secured to the lower LAL surface 50 or the patient contacting surface 52 opposite the inlet 44.

The three-dimensionally engineered spacer 48 can be positioned within the LAL chamber 42 and can separate the lower LAL surface 50 from the patient contacting surface 52. The three-dimensionally engineered spacer 48 can be composed of SpaceNet®, which is a product of Freudenberg. It should be appreciated that the three-dimensionally engineered spacer 48 can be composed of other materials having a high fluid porosity and having some resistance against flattening. It should also be appreciated that the three-dimensionally engineered spacer 42 can include at least one chamber and/or bladder (not shown) therewithin. It should also be appreciated that the mattress spacer 30 and the three-dimensionally engineered spacer 48 can be composed of the same material and have the same physical characteristics.

The resistance to flow for the three-dimensionally engineered spacer 48 when fluid is supplied to the LAL topper 18 at a rate of 2.2 ft³/min. can be less than about 1 (lb./in²)/(ft³/min.). In one illustrative embodiment, the resistance to flow for the three-dimensionally engineered spacer 48 can be between about .1 (lbs./in²)/(ft³/min.) and .7 (lbs./in²)/(ft³/min.). It should be appreciated that the resistance to flow can be more than 1 (lb./in²)/(ft³/min.). It should be appreciated that the moisture vapor transfer rate can be between 25 g/m²-hr and 200 g/m²-hr. Where three-dimensionally engineered spacer 48 is composed of SpaceNet®, the thickness of the three-dimensionally engineered spacer 48 can be between about .1 in. and .75 in. It should be appreciated that the thickness of the three-dimensionally engineered spacer 48 can be greater than .75 in.

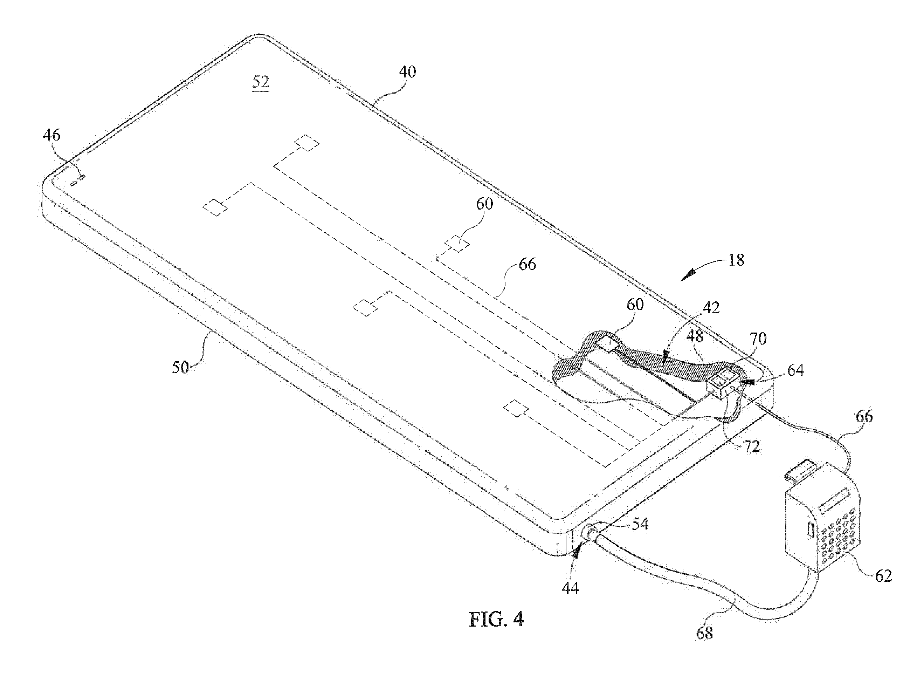

The control system 14 can include a plurality of sensors 60, a fluid supply 62, and a controller 64 as shown in FIGs. 3 & 4. The sensors 60 can be temperature sensors and/or moisture sensors that can be adapted to generate signals corresponding to the temperature and relative humidity of the patient contacting surface 52. The sensors 60 can be positioned within the LAL ticking 40 and can be operatively coupled with the controller 64 via a wire 66. It should be appreciated that the sensors 60 can be coupled to the patient contacting surface 52 or coupled within the LAL chamber 42. It should also be appreciated that the sensors 60 can communicate wirelessly with the controller 64.

The fluid supply 62 can be an air blower 62 that can supply air to the LAL topper 18. It should be appreciated that the fluid supply 62 can supply a various other gasses and/or liquids. The fluid supply 62 can removably couple with the LAL topper 18 via a hose 68. It should be appreciated that the fluid supply 62 can connect directly to the LAL topper 18. It should also be appreciated that the blower can be integrated or partially integrated within the mattress 16 or LAL topper 18. The fluid supply 62 may also include a heating element (not shown) and/or cooling element (not shown) that can heat and/or cool the fluid being supplied.

Determining what characteristics the fluid supply 62 can have in order to meet the desired range(s) can depend on the physical characteristics of the materials included in the support device 12, as well as any material (not shown) positioned between the patient P1 and the support device 12. The fluid supply can be configured to supply fluid to the LAL topper 18 at a rate of 2.2 ft³/min. and at a temperature of about 18.3 °C (65 °F) to 29.4 °C (85 °F) so that the temperature of the fluid flowing directly beneath the LAL ticking 40 portion proximate the sacrum is about 29.4 °C (85 °F) to 35 °C (95 °F). It should be appreciated that the fluid supply 62 can supply fluid at a rate of 2.2 ft³/min. and at a temperature of about 18.3 °C (65 °F) to 29.4 °C (85 °F) to the mattress 16. It should further be appreciated that the fluid supply 62 can supply fluid at a temperature of about -3.9 °C (25 °F) to 29.4 °C (85 °F). It should also be appreciated that the fluid supply 62 can be required to supply fluid at a rate higher or lower than 2.2 ft³/min. and/or at a temperature lower than the aforementioned range based on the physical characteristics of the materials included in the support device 12 and positioned between the patient P1 and the support device 12.

The controller 64 can be operatively coupled to the fluid supply 62 and the sensors 60, and can be positioned within the LAL chamber 42 of the LAL topper 18 as shown in FIGs. 3 & 4. It should be appreciated that the controller 64 may be mounted to the mattress 16, a hospital be frame (not shown), a footboard (not shown), the fluid supply 62, or other support structures. It should also be appreciated that the controller 64 can be integrated into the fluid supply 62, a hospital bed control system (not shown), or other control systems configured to regulate the fluid supply 62.

The controller 64 can include a processor 70 and memory 72 electrically coupled to the processor 70. The processor 70 can process the signals received from the sensors 60 and can execute instructions stored in the memory 72. The instructions can cause the controller 64 to regulate operation of the fluid supply 62 in accordance with the signals from the sensors 60 in order to maintain the temperature and/or relative humidity of the patient contacting surface 52 about the aforementioned ranges. The controller 64 can also receive a user input signal from a user input device (not shown) that allows the patient P1 to influence the regulation of the fluid supply 62. It should be appreciated that the user input signal can only influence the regulation of the fluid supply 62 within the aforementioned temperature and/or relative humidity ranges.

In operation, the fluid supply 62 can be initiated and fluid can be supplied through the hose 66 to the inlet 44 of the LAL topper 18. The fluid can flow into the LAL chamber 42, through the three-dimensionally engineered spacer 48, and out the vent 46. As the fluid flows through the LAL topper 18, the temperature and relative humidity of the patient contacting surface 52 can be maintained within the predetermined temperature and relative humidity ranges, such as, the ranges described earlier. The sensors 60 can generate signals representative of the temperature and relative humidity of the patient contact surface 52 and communicate the signals to the processor 70 of the controller 64. The processor 70 can process the signals and can execute the instructions stored in the memory 72 in accordance with the signals from the sensors 60 to regulate operation of the fluid supply 62 in accordance with the signals. It should be appreciated that a patient P1 need not be contacting the support device 12 for regulation of the temperature and relative humidity of the patient contacting surface 52 to occur.

Many other embodiments of the present disclosure are also envisioned. The structure of such embodiments may utilize one or more illustrative components described above, or other appropriate components, now know to be developed

For example, a microclimate management system 10 comprises a support device 12, a fluid supply 62, and a controller 64. The support device 12 includes a person contacting surface 52. The fluid supply 62 couples with the support device 12 and supplies fluid to the support device 12. The controller 64 is operatively coupled to the fluid supply 62 and includes an instruction set. The instruction set causes the controller 64 to regulate at least one of the rate the fluid is supplied by the fluid supply 62 and temperature of the fluid supplied by the fluid supply 62 to maintain a heat withdrawal capacity of at least a portion of the person contacting surface 52 below about 140 W/m²..

In another example, a microclimate management system 10 comprises a mattress 16, a topper 18, and a control system 14. The topper 18 is coupled to the mattress 16 and is supported thereon. The topper 18 defines an interior region 42 and a person contacting surface 52. The control system 14 is configured to maintain at least one of a surface temperature, a relative surface humidity, and a heat withdrawal capacity of at least a portion of the person contacting surface 52 within a predetermined operating range.

In another example, a microclimate management system 10 comprises a support device 12, a fluid supply 62, and a controller 64. The support device 12 includes a person contacting surface 52. The fluid supply 62 is coupled with the support device 12 and supplies fluid to the support device 12. The controller 64 includes an instruction set. The instruction set causes the controller 64 to regulate at least one of the rate the fluid is supplied by the fluid supply 62 and temperature of the fluid supplied by the fluid supply 62 to maintain a surface temperature of at least a portion of the person contacting surface 52 above about 88° Fahrenheit and a relative humidity of at least a portion of the person contacting surface 52 below about 95%.

In yet another example, at least one of a relative humidity and a temperature of at least a portion of a person contacting surface 52 of a topper 18 coupled on a mattress 16 is sensed. A fluid supply 62 in communication with the topper 18 is regulated as a function of at least one of the temperature of at least a portion of the person contacting surface 52 and the relative humidity of at least a portion of the person contacting surface 52 to maintain at least a portion of the person contacting surface 52 within a predetermined operating range.

In yet another example, at least one of a relative humidity and a temperature of at least a portion of a person contacting surface 52 of a support device 12 is sensed. A person comfort input signal is received from an input device 60. A a fluid supply 62 in communication with the support device 12 is regulated as a function of at least two of the temperature of at least a portion of the person contacting surface 52, the relative humidity of at least a portion of the person contacting surface 52, and the person comfort input signal to maintain at least a portion of the person contacting surface 52 within a predetermined operating range.

In still another example, at least one of a relative humidity and a temperature of at least a portion of a person contacting surface 52 of a topper 18 coupled on a mattress 16 is sensed. A person comfort input signal is received from an input device 60. A fluid supply 62 in communication with the topper 18 is regulated as a function of at least one of the temperature of at least a portion of the person contacting surface 52, the relative humidity of at least a portion of the person contacting surface 52, and the person comfort input signal to maintain at least a portion of the person contacting surface 52 within a predetermined operating range.

In still another example, a relative humidity and a temperature of a person contacting surface 52 of a support device 12 is sensed. At least one of a rate and a temperature of fluid supplied by a fluid supply 62 to the support device 12 is regulated as a function of the temperature of the person contacting surface 52 and the relative humidity of the person contacting surface 52 to maintain the person contacting surface 52 within a predetermined operating range.

In a further example, an apparatus comprises a mattress 16, a topper 18, and a sensor 60. The topper 18 is coupled on the mattress 16. The topper 18 includes a cover 40 and has fluid communicated through at least a portion of the topper 18. The topper 18 defines an interior region 42 and a portion of the cover 40 defines a person contacting surface 52. The sensor 60 is configured to sense at least one of a temperature of the person contacting surface 52 a relative humidity of the person contacting surface 52, a temperature of the fluid within the topper 18, and a relative humidity of the fluid within the topper 18.

In a further example, a support device 12 is provided. The support device 12 includes a cover 40and a spacer 48. The cover 40 defines an interior region 42 and at least a portion of the cover 40 defines a person contacting surface 52. The cover 40 also defines a fluid permeability parameter, a thermal conductivity parameter, and a thickness parameter. The spacer 48 is positioned within the interior region 42. The spacer 48 defines a fluid resistance parameter, a fluid permeability parameter, a thermal conductivity parameter, and a thickness parameter. A fluid supply 62 is selected to cooperate with at least one of the fluid permeability parameter of the cover 40, the thickness parameter of the cover 40, the thermal conductivity of the cover 40, the fluid resistance parameter of the spacer 48, the fluid permeability parameter of the spacer 48, the thermal conductivity parameter of the spacer 48, and the thickness parameter of the spacer 48 to maintain a heat withdrawal capacity of at least a portion of the person contacting surface 52 below about 140 W/m².

In yet a further example, a support device 12 is provided. The support device 12 includes a cover 40 and a spacer 48. The cover 40 defines an interior region 42 and at least a portion of the cover 40 defines a person contacting surface 52. The cover 40 also defines a fluid permeability parameter, a thermal conductivity parameter, and a thickness parameter. The spacer 48 is positioned within the interior region 42. The spacer 48 defines a fluid resistance parameter, a fluid permeability parameter, a thermal conductivity parameter, and a thickness parameter. At least one of the fluid permeability parameter of the cover 40, the thickness parameter of the cover 40, the thermal conductivity of the cover 40, the fluid resistance parameter of the spacer 48, the fluid permeability parameter of the spacer 48, the thermal conductivity parameter of the spacer 48, and the thickness parameter of the spacer 48 is varied to cooperate with the fluid supply 62 to maintain a heat withdrawal capacity of at least a portion of the person contacting surface 52 below about 140 W/m².

Any theory, mechanism of operation, proof, or finding stated herein is meant to further enhance understanding of the present disclosure and is not intended to make the present disclosure in any way dependent upon such theory, mechanism of operation, proof, or finding. It should be understood that while the use of the word preferable, preferably or preferred in the description above indicates that the feature so described may be more desirable, it nonetheless may not be necessary and embodiments lacking the same may be contemplated as within the scope of the disclosure, that scope being defined by the claims that follow. In reading the claims it is intended that when words such as "a," "an," "at least one," "at least a portion" are used there is no intention to limit the claim to only one item unless specifically stated to the contrary in the claim. When the language "at least a portion" and/or "a portion" is used the item may include a portion and/or the entire item unless specifically stated to the contrary. While the invention has been illustrated and described in detail in the drawings and foregoing description, the same is to be considered as illustrative and not restrictive in character, it being understood that only the selected embodiments have been shown and described and that all changes, modifications and equivalents that come within the spirit of the invention as defined herein or by any of the following claims are desired to be protected.
Embodiments of the invention can be described with reference to the following numbered clauses, with preferred features laid out in the dependent clauses:
1. A microclimate management system 10 comprising:
   a support device 12 including a person contacting surface 52;
   a fluid supply 62 coupled with the support device 12 and supplying fluid to the support device 12; and
   a controller 64 operatively coupled to the fluid supply 62 and including an instruction set, the instruction set causing the controller 64 to regulate at least one of the rate the fluid is supplied by the fluid supply 62 and temperature of the fluid supplied by the fluid supply 62 to maintain a heat withdrawal capacity of at least a portion of the person contacting surface 52 below about 140 W/m².
2. The microclimate management system 10 of clause 1, wherein the heat withdrawal capacity of at least a portion of the person contacting surface 52 is maintained above about 50 W/m².
3. The microclimate management system 10 of clause 1, wherein the heat withdrawal capacity of at least a portion of the person contacting surface 52 is maintained between about 60 W/m² and about 125 W/m².
4. The microclimate management system 10 of clause 1, wherein the temperature of the person contacting surface 52 is maintained below about 35.8 °C (96.5 °F).
5. The microclimate management system 10 of clause 1, wherein the temperature of the person contacting surface 52 is maintained above about 31.1 °C (88 °F).
6. The microclimate management system 10 of clause 1, wherein the temperature of the person contacting surface 52 is maintained between about 32.2 °C (90 °F) and about 35.3 °C (95.5 °F).
7. The microclimate management system 10 of clause 1, wherein the relative humidity of the person contacting surface 52 is maintained below about 95%.
8. The microclimate management system 10 of clause 1, wherein the relative humidity of the person contacting surface 52 is maintained above about 20%.
9. The microclimate management system 10 of clause 1, wherein the relative humidity of the person contacting surface 52 is maintained between about 55% and about 90%.
10. The microclimate management system 10 of clause 1 further comprising sensors 60 operatively coupled to the controller 64, the sensors 60 being configured to provide a signal indicative of one of the temperature and humidity of the person contacting surface 52.
11. The microclimate management system 10 of clause 10, wherein support device 12 includes a cover 40 defining an interior region 42 and a portion of the cover 40 defining the person contacting surface 52, the sensors 60 being integrated into the cover 40.
12. The microclimate management system 10 of clause 10, wherein the sensors 60 are positioned within the interior region 42 proximate the person contacting surface 52.
13. The microclimate management system 10 of clause 1, wherein the support device 12 includes at least one fluid bladder 28 therein, the controller 64 being configured to regulate the pressure of the fluid within the fluid bladder 28.
14. The microclimate management system 10 of clause 1, wherein the fluid supply 62 includes at least one of a heating element and a cooling element.
15. The microclimate management system 10 of clause 1, wherein the support device 12 includes a mattress 16 and a topper 18 coupled on the mattress 16, the topper 18 includes a cover 40 defining an interior region 42 and a portion of the cover 42 defining the person contacting surface 52.
16. The microclimate management system 10 of clause 15, wherein the topper18 includes a support layer 48 positioned within the interior region 42.
17. The microclimate management system 10 of clause 16, wherein the support layer 48 is a three-dimensionally engineered spacer.
18. The microclimate management system 10 of clause 1 further comprising an input device 60 configured to receive an input from a person indicative of a person's comfort level.
19. The microclimate management system 10 of clause 18, wherein the instruction set causes the controller 64 to further regulate the fluid supply 62 as a function of the input from the input device 60.
20. The microclimate management system 10 of clause 1, wherein the heat withdrawal capacity of at least a portion of the person contacting surface 52 is maintained below about 140 W/m² for less than about 6 hours.
21. The microclimate management system 10 of clause 1, wherein the heat withdrawal capacity of at least a portion of the person contacting surface 52 is maintained below about 140 W/m² for more than about 6 hours.
22. A microclimate management system 10 comprising:
   a mattress 16;
   a topper 18 coupled to the mattress 16 and supported thereon, the topper 18 defining an interior region 42 and a person contacting surface 52; and
   a control system 14 configured to maintain at least one of a surface temperature, a relative surface humidity, and a heat withdrawal capacity of at least a portion of the person contacting surface 52 within a predetermined operating range.
23. The microclimate management system 10 of clause 22, wherein the heat withdrawal capacity of at least a portion of the person contacting surface 52 is maintained below about 140 W/m².
24. The microclimate management system 10 of clause 22, wherein the heat withdrawal capacity of at least a portion of the person contacting surface 52 is maintained above about 50 W/m².
25. The microclimate management system 10 of clause 22, wherein the heat withdrawal capacity of at least a portion of the person contacting surface 52 is maintained between about 60 W/m² and about 125 W/m².
26. The microclimate management system 10 of clause 22, wherein the temperature of the person contacting surface 52 is maintained below about 35.8 °C (96.5 °F).
27. The microclimate management system 10 of clause 22, wherein the temperature of the person contacting surface 52 is maintained above about 31.1 °C (88 °F).
28. The microclimate management system 10 of clause 22, wherein the temperature of the person contacting surface 52 is maintained between about 32.2 °C (90 °F) and about 35.3 °C (95.5 °F).
29. The microclimate management system 10 of clause 22, wherein the relative humidity of the person contacting surface 52 is maintained below about 95%.
30. The microclimate management system 10 of clause 22, wherein the relative humidity of the person contacting surface 52 is maintained above about 20%.
31. The microclimate management system 10 of clause 22, wherein the relative humidity of the person contacting surface 52 is maintained between about 55% and about 90%.
32. The microclimate management system 10 of clause 22, wherein the control system 14 includes a controller 64 and sensors 60 operatively coupled to the controller 64, the sensors 60 being configured to provide a signal indicative of one of the temperature and humidity of the person contacting surface 52.
33. The microclimate management system 10 of clause 32, the sensors 60 are positioned within the interior region 42 proximate the person contacting surface 52.
34. The microclimate management system 10 of clause 32, wherein the sensors 60 are integrated into the person contacting surface 52.
35. The microclimate management system 10 of clause 22, wherein the topper18 includes a support layer 48 positioned within the interior region 42.
36. The microclimate management system 10 of clause 35, wherein the support layer 48 is a three-dimensionally engineered spacer.
37. The microclimate management system 10 of clause 35, wherein the support layer 48 includes at least one bladder 28.
38. The microclimate management system 10 of clause 35, wherein the support layer 48 is quilted.
39. The microclimate management system 10 of clause 22, wherein the control system 14 includes a fluid supply 62 configured to supply fluid to at least one of the topper 18 and the mattress 16.
40. The microclimate management system 10 of clause 39, wherein the fluid supply 62 includes at least one of a heating element and a cooling element.
41. The microclimate management system 10 of clause 39, wherein the control system 14 regulates at least one of the rate the fluid is supplied by the fluid supply 62 and temperature of the fluid supplied by the fluid supply 62 to maintain the person contacting surface 52 within the predetermined operating range.
42. The microclimate management system 10 of clause 22, wherein the control system 14 includes an input device 60 configured to receive input from a person indicative of a person's comfort level.
43. The microclimate management system 10 of clause 42, wherein the control system 14 further regulates the fluid supply 62 as a function of the input from the input device 60.
44. The microclimate management system of clause 22, wherein the topper 18 is secured to mattress 16 and supports a person on the person contacting surface 52.
45. The microclimate management system 10 of clause 22, wherein at least one of a surface temperature, a relative surface humidity, and a heat withdrawal capacity of at least a portion of the person contacting surface 52 is maintained within a predetermined operating range for less than about 6 hours.
46. The microclimate management system 10 of clause 22, wherein at least one of a surface temperature, a relative surface humidity, and a heat withdrawal capacity of at least a portion of the person contacting surface 52 is maintained within a predetermined operating range for more than about 6 hours.
47. A microclimate management system 10 comprising:
   a support device 12 including a person contacting surface 52;
   a fluid supply 62 coupled with the support device 12 and supplying fluid to the support device 12; and
   a controller 64 including an instruction set, the instruction set causing the controller 64 to regulate at least one of the rate the fluid is supplied by the fluid supply 62 and temperature of the fluid supplied by the fluid supply 62 to maintain a surface temperature of at least a portion of the person contacting surface 52 above about 88° Fahrenheit and a relative humidity of at least a portion of the person contacting surface 52 below about 95%.
48. The microclimate management system 10 of clause 47, wherein the heat withdrawal capacity of at least a portion of the person contacting surface 52 is maintained below about 140 W/m².
49. The microclimate management system 10 of clause 47, wherein the heat withdrawal capacity of at least a portion of the person contacting surface 52 is maintained above about 50 W/m².
50. The microclimate management system 10 of clause 47, wherein the heat withdrawal capacity of at least a portion of the person contacting surface 52 is maintained between about 60 W/m² and about 125 W/m².
51. The microclimate management system 10 of clause 47, wherein the temperature of the person contacting surface 52 is maintained below about 35.8 °C (96.5 °F).
52. The microclimate management system 10 of clause 47, wherein the temperature of the person contacting surface 52 is maintained between about 32.2 °C (90 °F) and about 35.3 °C (95.5 °F).
53. The microclimate management system 10 of clause 47, wherein the relative humidity of the person contacting surface 52 is maintained above about 20%.
54. The microclimate management system 10 of clause 47, wherein the relative humidity of the person contacting surface 52 is maintained between about 55% and about 90%.
55. The microclimate management system 10 of clause 47 further comprising sensors 60 operatively coupled to the controller 64, the sensors 60 being configured to provide a signal indicative of one of the temperature and humidity of the person contacting surface 52.
56. The microclimate management system 10 of clause 55, wherein support device 12 includes a cover 40, a portion of the cover 40 defining the person contacting surface 52, the sensors 60 being integrated into the person contacting surface 52.
57. The microclimate management system 10 of clause 56, wherein support device 12 includes a cover 40 defining an interior region 42, the sensors 60 being positioned within the interior region 42 proximate the person contacting surface 52.
58. The microclimate management system 10 of clause 47, wherein the support device 12 includes at least one fluid bladder 28 therein, the controller 64 being configured to regulate the pressure of the fluid within the fluid bladder 28.
59. The microclimate management system 10 of clause 47, wherein the fluid supply 62 includes at least one of a heating element and a cooling element.
60. The microclimate management system 10 of clause 47, wherein the support device 12 includes a mattress 16 and a topper 18 coupled on the mattress 16, the topper 18 includes a cover 40 defining an interior region 42 and a portion of the cover 42 defining the person contacting surface 52.
61. The microclimate management system 10 of clause 60, wherein the topper18 includes a support layer 48 positioned within the interior region 42.
62. The microclimate management system 10 of clause 61, wherein the support layer 48 is a three-dimensionally engineered spacer.
63. The microclimate management system 10 of clause 47 further comprising an input device 60 configured to receive an input from a person indicative of a person's comfort level.
64. The microclimate management system 10 of clause 47, wherein the instruction set causes the controller 64 to further regulate the fluid supply 62 as a function of the input from the input device 60.
65. The microclimate management system 10 of clause 47, wherein the surface temperature of at least a portion of the person contacting surface 52 is maintained above about 88° Fahrenheit and the relative humidity of at least a portion of the person contacting surface 52 is maintained below about 95% for more than about 6 hours.
66. The microclimate management system 10 of clause 47, wherein the surface temperature of at least a portion of the person contacting surface 52 is maintained above about 88° Fahrenheit and the relative humidity of at least a portion of the person contacting surface 52 is maintained below about 95% for less than about 6 hours.
67. A method comprising:
   sensing at least one of a relative humidity and a temperature of at least a portion of a person contacting surface 52 of a topper 18 coupled on a mattress 16; and
   regulating a fluid supply 62 in communication with the topper 18 as a function of at least one of the temperature of at least a portion of the person contacting surface 52 and the relative humidity of at least a portion of the person contacting surface 52 to maintain at least a portion of the person contacting surface 52 within a predetermined operating range.
68. The method of clause 67 further comprising the steps of:
   sensing the temperature of the area proximate one of the mattress 16 and the topper 18;
   calculating a heat withdrawal capacity of at least a portion of the person contacting surface 52 as a function of the temperature of at least a portion of the contacting surface 52, the relative humidity of at least a portion of the contacting surface 52, and the temperature of an area proximate one of the mattress 16 and the topper 18; and
   further regulating the fluid supply 68 as a function of the heat withdrawal capacity to maintain to maintain at least a portion of the person contacting surface 52 within the predetermined operating range.
69. The method of clause 68, wherein the heat withdrawal capacity of at least a portion of the person contacting surface 52 is maintained below about 140 W/m².
70. The method of clause 68, wherein the heat withdrawal capacity of at least a portion of the person contacting surface 52 is maintained above about 50 W/m².
71. The method of clause 68, wherein the heat withdrawal capacity of at least a portion of the person contacting surface 52 is maintained between about 60 W/m² and about 125 W/m².
72. The method of clause 67, wherein the temperature of the person contacting surface 52 is maintained below about 35.8 °C (96.5 °F).
73. The method of clause 67, wherein the temperature of the person contacting surface 52 is maintained above about 31.1 °C (88 °F).
74. The method of clause 67, wherein the temperature of the person contacting surface 52 is maintained between about 32.2 °C (90 °F) and about 35.3 °C (95.5 °F).
75. The method of clause 67, wherein the relative humidity of the person contacting surface 52 is maintained below about 95%.
76. The method of clause 67, wherein the relative humidity of the person contacting surface 52 is maintained above about 20%.
77. The method of clause 67, wherein the relative humidity of the person contacting surface 52 is maintained between about 55% and about 90%.
78. The method of clause 67, wherein the at least one of a relative humidity and a temperature of at least a portion of a person contacting surface 52 is sensed by sensors 60.
79. The method of clause 78, wherein the sensors 60 are positioned proximate the person contacting surface 52.
80. The method of clause 78, wherein the sensors 60 are integrated into the person contacting surface 52.
81. The method of clause 67, wherein the topper18 includes a cover defining an interior region 42 and a support layer 48 positioned within the interior region 42.
82. The method of clause 81, wherein the support layer 48 is a three-dimensionally engineered spacer.
83. The method of clause 81, wherein the support layer 48 includes at least one bladder 28.
84. The method of clause 81, wherein the support layer 48 is quilted.
85. The method of clause 67, wherein the fluid supply 62 includes at least one of a heating element and a cooling element.
86. The method of clause 67, wherein at least one of the rate the fluid is supplied by the fluid supply 62 and temperature of the fluid supplied by the fluid supply 62 is regulated to maintain the person contacting surface 52 within the predetermined operating range.
87. The method of clause 67, wherein the fluid supply 62 is further regulated as a function of a user input indicative of a person's comfort level input through an input device 60.
88. The method of clause 67, wherein the at least a portion of the person contacting surface is maintained within the predetermined operating range for less than about 6 hours.
89. The method of clause 67, wherein the at least a portion of the person contacting surface is maintained within the predetermined operating range for more than about 6 hours.
90. A method comprising:
   sensing at least one of a relative humidity and a temperature of at least a portion of a person contacting surface 52 of a support device 12;
   receiving a person comfort input signal from an input device 60; and
   regulating a fluid supply 62 in communication with the support device 12 as a function of at least two of the temperature of at least a portion of the person contacting surface 52, the relative humidity of at least a portion of the person contacting surface 52, and the person comfort input signal to maintain at least a portion of the person contacting surface 52 within a predetermined operating range.
91. The method of clause 90 further comprising the steps of:
   sensing the temperature of the area proximate the support device 12;
   calculating a heat withdrawal capacity of at least a portion of the person contacting surface 52 as a function of the temperature of at least a portion of the contacting surface 52, the relative humidity of at least a portion of the contacting surface 52, and the temperature of an area proximate the support device 12; and
   further regulating the fluid supply 62 as a function of the heat withdrawal capacity to maintain to maintain at least a portion of the person contacting surface 52 within the predetermined operating range.
92. The method of clause 91, wherein the heat withdrawal capacity of at least a portion of the person contacting surface 52 is maintained below about 140 W/m².
93. The method of clause 91, wherein the heat withdrawal capacity of at least a portion of the person contacting surface 52 is maintained above about 50 W/m².
94. The method of clause 91, wherein the heat withdrawal capacity of at least a portion of the person contacting surface 52 is maintained between about 60 W/m² and about 125 W/m².
95. The method of clause 90, wherein the temperature of the person contacting surface 52 is maintained below about 35.8 °C (96.5 °F).
96. The method of clause 90, wherein the temperature of the person contacting surface 52 is maintained above about 31.1 °C (88 °F).
97. The method of clause 90, wherein the temperature of the person contacting surface 52 is maintained between about 32.2 °C (90 °F) and about 35.3 °C (95.5 °F).
98. The method of clause 90, wherein the relative humidity of the person contacting surface 52 is maintained below about 95%.
99. The method of clause 90, wherein the relative humidity of the person contacting surface 52 is maintained above about 20%.
100. The method of clause 90, wherein the relative humidity of the person contacting surface 52 is maintained between about 55% and about 90%.
101. The method of clause 90, wherein the at least one of a relative humidity and a temperature of at least a portion of a person contacting surface 52 is sensed by sensors 60.
102. The method of clause 101, wherein the sensors 60 are positioned proximate the person contacting surface 52.
103. The method of clause 101, wherein the sensors 60 are integrated into the person contacting surface 52.
104. The method of clause 90, wherein the support device includes a mattress 16 and a topper 18 coupled on the mattress 16, the topper 18 having a cover 40 defining an interior region 42 and a support layer 48 positioned within the interior region 42.
105. The method of clause 104, wherein the support layer 48 is a three-dimensionally engineered spacer.
106. The method of clause 90, wherein the fluid supply 62 includes at least one of a heating element and a cooling element.
107. The method of clause 90, wherein at least one of the rate the fluid is supplied by the fluid supply 62 and temperature of the fluid supplied by the fluid supply 62 is regulated to maintain the person contacting surface 52 within the predetermined operating range.
108. The method of clause 90, wherein the input device 60 is a touch screen input device.
109. The method of clause 90, wherein the input device 60 is a keyboard input device.
110. The method of clause 90, wherein the input device 60 is a pendant input device.
111. The method of clause 90, wherein the at least a portion of the person contacting surface is maintained within the predetermined operating range for less than about 6 hours.
112. The method of clause 90, wherein the at least a portion of the person contacting surface is maintained within the predetermined operating range for more than about 6 hours.
113. A method comprising:
   sensing at least one of a relative humidity and a temperature of at least a portion of a person contacting surface 52 of a topper 18 coupled on a mattress 16;
   receiving a person comfort input signal from an input device 60; and
   regulating a fluid supply 62 in communication with the topper 18 as a function of at least one of the temperature of at least a portion of the person contacting surface 52, the relative humidity of at least a portion of the person contacting surface 52, and the person comfort input signal to maintain at least a portion of the person contacting surface 52 within a predetermined operating range.
114. The method of clause 113 further comprising the steps of:
   sensing the temperature of the area proximate the support device 12;
   calculating a heat withdrawal capacity of at least a portion of the person contacting surface 52 as a function of the temperature of at least a portion of the contacting surface 52, the relative humidity of at least a portion of the contacting surface 52, and the temperature of an area proximate the support device 12; and
   further regulating the fluid supply 62 as a function of the heat withdrawal capacity to maintain to maintain at least a portion of the person contacting surface 52 within the predetermined operating range.
115. The method of clause 114, wherein the heat withdrawal capacity of at least a portion of the person contacting surface 52 is maintained below about 140 W/m².
116. The method of clause 114, wherein the heat withdrawal capacity of at least a portion of the person contacting surface 52 is maintained above about 50 W/m².
117. The method of clause 114, wherein the heat withdrawal capacity of at least a portion of the person contacting surface 52 is maintained between about 60 W/m² and about 125 W/m².
118. The method of clause 113, wherein the temperature of the person contacting surface 52 is maintained below about 35.8 °C (96.5 °F).
119. The method of clause 113, wherein the temperature of the person contacting surface 52 is maintained above about 31.1 °C (88 °F).
120. The method of clause 113, wherein the temperature of the person contacting surface 52 is maintained between about 32.2 °C (90 °F) and about 35.3 °C (95.5 °F).
121. The method of clause 113, wherein the relative humidity of the person contacting surface 52 is maintained below about 95%.
122. The method of clause 113, wherein the relative humidity of the person contacting surface 52 is maintained above about 20%.
123. The method of clause 113, wherein the relative humidity of the person contacting surface 52 is maintained between about 55% and about 90%.
124. The method of clause 113, wherein the at least one of a relative humidity and a temperature of at least a portion of a person contacting surface 52 is sensed by sensors 60.
125. The method of clause 124, wherein the sensors 60 are positioned within the topper 18 proximate the person contacting surface 52.
126. The method of clause 124, wherein the sensors 60 are integrated into the person contacting surface 52.
127. The method of clause 113, wherein the topper 18 has a cover 40 defining an interior region 42 and a support layer 48 positioned within the interior region 42.
128. The method of clause 127, wherein the support layer 48 is a three-dimensionally engineered spacer.
129. The method of clause 127, wherein the support layer 48 includes at least one bladder 28.
130. The method of clause 127, wherein the support layer 48 is quilted.
131. The method of clause 113, wherein the fluid supply 62 includes at least one of a heating element and a cooling element.
132. The method of clause 113, wherein the user input device 60 is a touch screen input device.
133. The method of clause 113, wherein the user input device 60 is a keyboard input device.
134. The method of clause 113, wherein the user input device 60 is a pendant input device.
135. The method of clause 113, wherein at least one of the rate the fluid is supplied by the fluid supply 62 and temperature of the fluid supplied by the fluid supply 62 is regulated to maintain the person contacting surface 52 within the predetermined operating range.
136. The method of clause 113, wherein the at least a portion of the person contacting surface is maintained within the predetermined operating range for less than about 6 hours.
137. The method of clause 113, wherein the at least a portion of the person contacting surface is maintained within the predetermined operating range for more than about 6 hours.
138. A method comprising:
   sensing a relative humidity and a temperature of a person contacting surface 52 of a support device 12; and
   regulating at least one of a rate and a temperature of fluid supplied by a fluid supply 62 to the support device 12 as a function of the temperature of the person contacting surface 52 and the relative humidity of the person contacting surface 52 to maintain the person contacting surface 52 within a predetermined operating range.
139. The method of clause 138 further comprising the steps of:
   sensing the temperature of the area proximate one of the support device 12;
   calculating a heat withdrawal capacity of at least a portion of the person contacting surface 52 as a function of the temperature of at least a portion of the contacting surface 52, the relative humidity of at least a portion of the contacting surface 52, and the temperature of an area proximate the support device 12; and
   further regulating the fluid supply 62 as a function of the heat withdrawal capacity to maintain to maintain at least a portion of the person contacting surface 52 within the predetermined operating range.
140. The method of clause 139, wherein the heat withdrawal capacity of at least a portion of the person contacting surface 52 is maintained below about 140 W/m².
141. The method of clause 139, wherein the heat withdrawal capacity of at least a portion of the person contacting surface 52 is maintained above about 50 W/m².
142. The method of clause 139, wherein the heat withdrawal capacity of at least a portion of the person contacting surface 52 is maintained between about 60 W/m² and about 125 W/m².
143. The method of clause 138 , wherein the temperature of the person contacting surface 52 is maintained below about 35.8 °C (96.5 °F).
144. The method of clause 138 , wherein the temperature of the person contacting surface 52 is maintained above about 31.1 °C (88 °F).
145. The method of clause 138 , wherein the temperature of the person contacting surface 52 is maintained between about 32.2 °C (90 °F) and about 35.3 °C (95.5 °F).
146. The method of clause 138 , wherein the relative humidity of the person contacting surface 52 is maintained below about 95%.
147. The method of clause 138 , wherein the relative humidity of the person contacting surface 52 is maintained above about 20%.
148. The method of clause 138 , wherein the relative humidity of the person contacting surface 52 is maintained between about 55% and about 90%.
149. The method of clause 138 , wherein the at least one of a relative humidity and a temperature of at least a portion of a person contacting surface 52 is sensed by sensors 60.
150. The method of clause 149, wherein the sensors 60 are positioned proximate the person contacting surface 52.
151. The method of clause 149, wherein the sensors 60 are integrated into the person contacting surface 52.
152. The method of clause 138, wherein the support device 12 includes a mattress 16 and a topper 18 coupled on the mattress 16, the topper 18 includes a cover 40 that defines an interior region 42.
153. The method of clause 152, wherein the topper 18 includes a support layer 48 positioned within the interior region 42.
154. The method of clause 153, wherein the support layer 48 is a three-dimensionally engineered spacer.
155. The method of clause 138, wherein the fluid supply 62 includes at least one of a heating element and a cooling element.
156. The method of clause 138, wherein the fluid supply 62 is further regulated as a function of a user input indicative of a person's comfort level input through a user input device 60.
157. The method of clause 138, wherein the at least a portion of the person contacting surface is maintained within the predetermined operating range for less than about 6 hours.
158. The method of clause 138, wherein the at least a portion of the person contacting surface is maintained within the predetermined operating range for more than about 6 hours.
159. An apparatus comprising:
   a mattress 16;
   a topper 18 coupled on the mattress 16, the topper 18 including a cover 40 and having fluid communicated through at least a portion of the topper 18, the topper 18 defining an interior region 42 and a portion of the cover 40 defining a person contacting surface 52; and
   a sensor 60 configured to sense at least one of a temperature of the person contacting surface 52 a relative humidity of the person contacting surface 52, a temperature of the fluid within the topper 18, and a relative humidity of the fluid within the topper 18.
160. The apparatus of clause 159, wherein the sensors 60 are positioned within the topper 18 proximate the person contacting surface 52.
161. The apparatus of clause 159, wherein the sensors 60 are integrated into the person contacting surface 52.
162. The apparatus of clause 159, wherein the cover 40 defines an interior region 42, the topper 18 including a support layer 48 positioned within the interior region 42.
163. The apparatus of clause 162, wherein the support layer 48 is a three-dimensionally engineered spacer.
164. The apparatus of clause 162, wherein the support layer includes at least one bladder.
165. The apparatus of clause 162, wherein the support layer is quilted.
166. A method of manufacturing a microclimate management system 10 comprising:
   providing a support device 12, the support device 12 including:
      a cover 40 defining an interior region 42 and at least a portion of the cover 40 defining a person contacting surface 52, the cover 40 also defining a fluid permeability parameter, a thermal conductivity parameter, and a thickness parameter, and
      a spacer 48 positioned within the interior region 42, the spacer 48 defining a fluid resistance parameter, a fluid permeability parameter, a thermal conductivity parameter, and a thickness parameter; and
      selecting a fluid supply 62 to cooperate with at least one of the fluid permeability parameter of the cover 40, the thickness parameter of the cover 40, the thermal conductivity of the cover 40, the fluid resistance parameter of the spacer 48, the fluid permeability parameter of the spacer 48, the thermal conductivity parameter of the spacer 48, and the thickness parameter of the spacer 48 to maintain a heat withdrawal capacity of at least a portion of the person contacting surface 52 below about 140 W/m².
167. The method of clause 166, wherein the fluid supply 62 is configured to supply fluid at a rate of about 2.2 ft³/min.
168. The method of clause 166, wherein the fluid supply 62 is configured to supply fluid at a temperature of about -3.9 °C (25 °F) to about 29.4 °C (85 °F).
169. The method of clause 166, wherein the fluid supply 62 is configured to supply fluid at a temperature of about 18.3 °C (65 °F) to about 29.4 °C (85 °F).
170. The method of clause 166, wherein the fluid resistance parameter of the spacer 48 is less than about 1 (lb./in²)/(ft³/min.) when fluid is supplied at a rate of about 2.2 ft³/min.
171. The method of clause 166, wherein the fluid resistance parameter of the spacer 48 is greater than about 1 (lb./in²)/(ft³/min.) when fluid is supplied at a rate of about 2.2 ft³/min.
172. The method of clause 166, wherein the fluid resistance parameter of the spacer 48 is between about1 (lbs./in²)/(ft³/min.) and .about 7 (lbs./in²)/(ft³/min.) when fluid is supplied at a rate of about 2.2 ft³/min.
173. The method of clause 166, wherein the fluid permeability parameter of the spacer 48 is between about 25 g/m²-hr and about 200 g/m²-hr when fluid is supplied at a rate of about 2.2 ft³/min.
174. The method of clause 166, wherein the thickness of the spacer 48 is between about.1 in. and about .75 in.
175. The method of clause 166, wherein the fluid permeability parameter of the cover 40 is at least 20 g/m²-hr when fluid is supplied at a rate of about 2.2 ft³/min.
176. The method of clause 166, wherein the fluid permeability parameter of the cover 40 is at least 80 g/m²-hr when fluid is supplied at a rate of about 2.2 ft³/min.
177. The method of clause 166, wherein the fluid permeability parameter of the cover 40 is between about 25 g/m²-hr and about 200 g/m²-hr. when fluid is supplied at a rate of about 2.2 ft³/min.
178. The method of clause 166, wherein the fluid supply 62 is selected to further cooperate with a material positioned between a person and the person contacting surface 52, the material having a thermal conductivity of between about .02 W/m - K and about 1000 W/m - K.
179. The method of clause 166, wherein the heat withdrawal capacity of at least a portion of the person contacting surface 52 is maintained above about 50 W/m².
180. The method of clause 166, wherein the heat withdrawal capacity of at least a portion of the person contacting surface 52 is maintained between about 60 W/m² and about 125 W/m².
181. The method of clause 166, wherein the temperature of the person contacting surface 52 is maintained below about 35.8 °C (96.5 °F).
182. The method of clause 166, wherein the temperature of the person contacting surface 52 is maintained above about 31.1 °C (88 °F).
183. The method of clause 166, wherein the temperature of the person contacting surface 52 is maintained between about 32.2 °C (90 °F) and about 35.3 °C (95.5 °F).
184. The method of clause 166, wherein the relative humidity of the person contacting surface 52 is maintained below about 95%.
185. The method of clause 166, wherein the relative humidity of the person contacting surface 52 is maintained above about 20%.
186. The method of clause 166, wherein the relative humidity of the person contacting surface 52 is maintained between about 55% and about 90%.
187. The method of clause 166, wherein at least one of a relative humidity and a temperature of at least a portion of a person contacting surface 52 is sensed by sensors 60.
188. The method of clause 187, wherein the sensors 60 are positioned proximate the person contacting surface 52.
189. The method of clause 187, wherein the sensors 60 are integrated into the person contacting surface 52.
190. The method of clause 166, wherein the support device 12 includes a mattress 16 and a topper 18 coupled on the mattress 16, the topper 18 includes a cover 40 that defines an interior region 42.
191. The method of clause 190, wherein the topper 18 includes a support layer 48 positioned within the interior region 42.
192. The method of clause 191, wherein the support layer 48 is a three-dimensionally engineered spacer.
193. The method of clause 166, wherein the fluid supply 62 includes at least one of a heating element and a cooling element.
194. The method of clause 166, wherein the fluid supply 62 is further regulated as a function of a user input indicative of a person's comfort level input through an input device 60.
195. The method of clause 166, wherein the support device 12 further includes a bladder 28 positioned within the interior region 42 and defining a fluid permeability parameter, a thermal conductivity parameter, and a thickness parameter, selecting a fluid supply 62 to further cooperate with at least one of the fluid permeability parameter of the bladder 28, the thickness parameter of the bladder 28, and the thermal conductivity of the bladder 28.
196. The method of clause 166, wherein a heat withdrawal capacity of at least a portion of the person contacting surface 52 is maintained below about 140 W/m² for less than about 6 hours.
197. The method of clause 166, wherein a heat withdrawal capacity of at least a portion of the person contacting surface 52 is maintained below about 140 W/m² for more than about 6 hours.
198. A method of manufacturing a microclimate management system 10, comprising:
   providing a support device 12 in communication with a fluid supply 62, the support device 12 including:
   a cover 40 defining an interior region 42 and at least a portion of the cover 40 defining a person contacting surface 52, the cover 40 also defining a fluid permeability parameter, a thermal conductivity parameter, and a thickness parameter, and
   a spacer 48 positioned within the interior region 42, the spacer 48 defining a fluid resistance parameter, a fluid permeability parameter, a thermal conductivity parameter, and a thickness parameter; and
   varying at least one of the fluid permeability parameter of the cover 40, the thickness parameter of the cover 40, the thermal conductivity of the cover 40, the fluid resistance parameter of the spacer 48, the fluid permeability parameter of the spacer 48, the thermal conductivity parameter of the spacer 48, and the thickness parameter of the spacer 48 to cooperate with the fluid supply 62 to maintain a heat withdrawal capacity of at least a portion of the person contacting surface 52 below about 140 W/m².
199. The method of clause 198, wherein the fluid supply 62 is configured to supply fluid at a rate of about 2.2 ft³/min.
200. The method of clause 198, wherein the fluid supply 62 is configured to supply fluid at a temperature of about -3.9 °C (25 °F) to about 29.4 °C (85 °F).
201. The method of clause 198, wherein the fluid supply 62 is configured to supply fluid at a temperature of about 18.3 °C (65 °F) to about 29.4 °C (85 °F).
202. The method of clause 198, wherein the fluid resistance parameter of the spacer 48 is less than about 1 (lb./in²)/(ft³/min.) when fluid is supplied at a rate of about 2.2 ft³/min.
203. The method of clause 198, wherein the fluid resistance parameter of the spacer 48 is greater than about 1 (lb./in²)/(ft³/min.) when fluid is supplied at a rate of about 2.2 ft³/min.
204. The method of clause 198, wherein the fluid resistance parameter of the spacer 48 is between about1 (lbs./in²)/(ft³/min.) and .about 7 (lbs./in²)/(ft³/min.) when fluid is supplied at a rate of about 2.2 ft³/min.
205. The method of clause 198, wherein the fluid permeability parameter of the spacer 48 is between about 25 g/m²-hr and about 200 g/m²-hr when fluid is supplied at a rate of about 2.2 ft³/min.
206. The method of clause 198, wherein the thickness of the spacer 48 is between about .1 in. and about .75 in.
207. The method of clause 198, wherein the fluid permeability parameter of the cover 40 is at least 20 g/m²-hr when fluid is supplied at a rate of about 2.2 ft³/min.
208. The method of clause 198, wherein the fluid permeability parameter of the cover 40 is at least 80 g/m²-hr when fluid is supplied at a rate of about 2.2 ft³/min.
209. The method of clause 198, wherein the fluid permeability parameter of the cover 40 is between about 25 g/m²-hr and about 200 g/m²-hr. when fluid is supplied at a rate of about 2.2 ft³/min.
210. The method of clause 198, wherein the fluid supply 62 is selected to further cooperate with a material positioned between a person and the person contacting surface 52, the material having a thermal conductivity of between about .02 W/m - K and about 1000 W/m - K.
211. The method of clause 198, wherein the heat withdrawal capacity of at least a portion of the person contacting surface 52 is maintained above about 50 W/m².
212. The method of clause 198, wherein the heat withdrawal capacity of at least a portion of the person contacting surface 52 is maintained between about 60 W/m² and about 125 W/m².
213. The method of clause 198, wherein the temperature of the person contacting surface 52 is maintained below about 35.8 °C (96.5 °F).
214. The method of clause 198, wherein the temperature of the person contacting surface 52 is maintained above about 31.1 °C (88 °F).
215. The method of clause 198, wherein the temperature of the person contacting surface 52 is maintained between about 32.2 °C (90 °F) and about 35.3 °C (95.5 °F).
216. The method of clause 198, wherein the relative humidity of the person contacting surface 52 is maintained below about 95%.
217. The method of clause 198, wherein the relative humidity of the person contacting surface 52 is maintained above about 20%.
218. The method of clause 198, wherein the relative humidity of the person contacting surface 52 is maintained between about 55% and about 90%.
219. The method of clause 198, wherein at least one of a relative humidity and a temperature of at least a portion of a person contacting surface 52 is sensed by sensors 60.
220. The method of clause 219, wherein the sensors 60 are positioned proximate the person contacting surface 52.
221. The method of clause 219, wherein the sensors 60 are integrated into the person contacting surface 52.
222. The method of clause 198, wherein the support device 12 includes a mattress 16 and a topper 18 coupled on the mattress 16, the topper 18 includes a cover 40 that defines an interior region 42.
223. The method of clause 222, wherein the topper 18 includes a support layer 48 positioned within the interior region 42.
224. The method of clause 223, wherein the support layer 48 is a three-dimensionally engineered spacer.
225. The method of clause 198, wherein the fluid supply 62 includes at least one of a heating element and a cooling element.
226. The method of clause 198, wherein the fluid supply 62 is further regulated as a function of a user input indicative of a person's comfort level input through an input device 60.
227. The method of clause 198, wherein the support device 12 further includes a bladder 28 positioned within the interior region 42 and defining a fluid permeability parameter, a thermal conductivity parameter, and a thickness parameter, further varying at least one of the fluid permeability parameter of the bladder 28, the thickness parameter of the bladder 28, and the thermal conductivity of the bladder 28 to cooperate with the fluid supply 62 to maintain a heat withdrawal capacity of at least a portion of the person contacting surface 52 below about 140 W/m²
228. The method of clause 198, wherein the heat withdrawal capacity of at least a portion of the person contacting surface 52 is maintained below about 140 W/m² for less than 6 hours.
229. The method of clause 198, wherein the heat withdrawal capacity of at least a portion of the person contacting surface 52 is maintained below about 140 W/m² for more than 6 hours.

## Claims

1. A method comprising:
sensing at least one of a relative humidity and a temperature of at least a portion of a person contacting surface (52) of a support device (12);
receiving a person comfort input signal from an input device (60); and
regulating a fluid supply (62) in communication with the support device (12) as a function of at least two of the temperature of at least a portion of the person contacting surface (52), the relative humidity of at least a portion of the person contacting surface (52), and the person comfort input signal to maintain at least a portion of the person contacting surface (52) within a predetermined operating range.

2. The method of claim 1 further comprising the steps of:
sensing the temperature of the area proximate the support device (12);
calculating a heat withdrawal capacity of at least a portion of the person contacting surface (52) as a function of the temperature of at least a portion of the contacting surface (52), the relative humidity of at least a portion of the contacting surface (52), and the temperature of an area proximate the support device (12); and
further regulating the fluid supply (62) as a function of the heat withdrawal capacity to maintain to maintain at least a portion of the person contacting surface (52) within the predetermined operating range.

3. The method of claim 2, wherein the heat withdrawal capacity of at least a portion of the person contacting surface (52) is maintained below about 140 W/m².

4. The method of claim 2, wherein the heat withdrawal capacity of at least a portion of the person contacting surface (52) is maintained above about 50 W/m².

5. The method of any preceding claim, wherein the temperature of the person contacting surface (52) is maintained below about 35.8 °C (96.5 °F).

6. The method of any preceding claim, wherein the temperature of the person contacting surface (52) is maintained above about 31.1 °C (88 °F).

7. The method of any preceding claim, wherein the relative humidity of the person contacting surface (52) is maintained below about 95%.

8. The method of any preceding claim, wherein the relative humidity of the person contacting surface (52) is maintained above about 20%.

9. The method of any preceding claim wherein the at least one of a relative humidity and a temperature of at least a portion of a person contacting surface (52) is sensed by sensors (60) positioned proximate the person contacting surface (52).

10. The method of any one of claims 1 to 8, wherein the at least one of a relative humidity and a temperature of at least a portion of a person contacting surface (52) is sensed by sensors (60) integrated into the person contacting surface (52).

11. The method of any preceding claim, wherein the support device includes a mattress (16) and a topper (18) coupled on the mattress (16), the topper (18) having a cover (40) defining an interior region (42) and a support layer (48) positioned within the interior region (42).

12. The method of claim 11, wherein the support layer (48) is a three-dimensionally engineered spacer.

13. The method of any preceding claim, wherein the fluid supply (62) includes at least one of a heating element and a cooling element.

14. The method of any preceding claim, wherein at least one of the rate the fluid is supplied by the fluid supply (62) and temperature of the fluid supplied by the fluid supply (62) is regulated to maintain the person contacting surface (52) within the predetermined operating range.

15. The method of any preceding claim, wherein the input device (60) is a pendant input device.
